# EUROPEAN PATENT SPECIFICATION

(11) **EP 2 977 383 B1**
(45) Date of publication and mention of the grant of the patent: **06.06.2018**
(21) Application number: 14178145.0
(22) Date of filing: 23.07.2014
(51) Int. Cl.: C07K 14/47, A61M 1/38

(54) **An in-vitro method and purification system for blood samples**
In-vitro-Verfahren und Reinigungssystem für Blutproben
Procédé in vitro et système de purification pour des échantillons de sang

(43) Date of publication of application: 27.01.2016
(73) Proprietor: Universitätsmedizin der Johannes Gutenberg-Universität Mainz, 55131 Mainz (DE)
(72) Inventor: Gabriel, Matthias, 55113 Mainz (DE); Holtappels, Rafaela, 55128 Mainz (DE)
(74) Representative: Patentanwälte Dr. Keller, Schwertfeger Partnerschaft mbB

(56) References cited:
- WO-A1-2013/039190
- JP-A- 2010 184 022
- ALLEN J. DUPLANTIER ET AL: "The Human Cathelicidin Antimicrobial Peptide LL-37 as a Potential Treatment for Polymicrobial Infected Wounds", FRONTIERS IN IMMUNOLOGY, vol. 4, 1 January 2013 (2013-01-01), XP055096533, DOI: 10.3389/fimmu.2013.00143

## Description

Blood samples or blood donations obtained from patients may be contaminated with various germs such as bacteria, yeasts or viruses, or pathogenic components thereof. Bacterial diseases such as sepsis often result from the persistence of microorganisms or their toxins in the blood stream. This is in particular relevant when microorganisms or their toxins are present in blood samples that are collected from patients for blood donations. Donated blood samples are usually tested for evidence of certain infectious disease pathogens, such as hepatitis B and C viruses, cytomegalo virus (CMV) and human immunodeficiency virus (HIV). The endotoxin lipopolysaccharide (LPS) is a major component of the outer cell membrane of gram-negative bacteria. It has been implicated in septic shock and multiple organ failure (Morrison D.C., Rian Y.L., Endotoxin and disease mechanism. Annu Rev Med 1987; 38:417-432). These diseases have an associated mortality rate of approximately 50 %. LPS remains active even when treatment with antibiotics is initiated.

Systematic screening of blood donations for the presence of blood-borne viruses has resulted in a dramatic decrease of viral infections after blood transfusion, and bacterial sepsis has become the most frequent infectious complication of transfusion in developed countries.

In this regards, preparations with red blood cell (RBC) are the most frequently transfused blood component for donations. Although contamination of platelet concentrates are predominantly attributable to gram-positive bacteria from the skin flora, contaminations of RBC products involve primarily gram-negative organisms (mostly members of the Enterobacteriaceae) of endogenous origin. Yersinia enterocolitica, associated with nearly 50% of documented cases of clinical sepsis from contaminated RBCs, is paradigmatic of such gram-negative agents transmitted by blood infusion and triggering severe sepsis and septic shock.

Antimicrobial peptides (AMP) are biological effectors of immediate defences in innate immunity. In mammals, they are included in the barrier protection of epithelia, where their expression can be induced during infection and inflammation (Stolzenberg ED, Anderson GM, Ackermann MR, et al., Epithelial antibiotic induced in states of disease. Proc Natl Acad Sci USA. 1997; 94:8686-8690; Froh M, Agerberth B, Ahangari G, et al., The expression of the gene coding for the antibacterial peptide LL-37 is induced in human keratinocytes during inflammatory disorders, J Biol Chem. 1997; 272:15258-1526). These peptides also constitute part of the non-oxidative bactericidal armament of phagocytes such as neutrophils and macrophages, and they can be secreted, as was shown for the precursor of LL-37 (hCAP18) (Sorensen O, Cowland JB, Askaa J., et al. An ELISA for hCAP-18, the cathelicidin present in human neutrophils and plasma. J Immunol Methods. 1997; 206:53-59). Several peptides with antimicrobial activity have been identified in humans. These include the α-defensins (HNP 1-4), which are mainly found in neutrophils (Lehrer RI, Lichtenstein AK, Ganz T. Defensins: antimicrobial and cytotoxic peptides of mammalian cells, Annu Rev Immunol. 1993; 11:105-128), and HD 5 and 6, which are expressed in Paneth cells of the small intestine (Jones DE, Bevins CL, Paneth cells of the human small intestine express an antimicrobial peptide gene, J Biol Chem. 1992;267:23216-23225; Jones DE, Bevins CL. Defensin-6 mRNA in human Paneth cells: implications for antimicrobial peptides in host defense of the human bowel, FEBS Lett. 1993; 315:187-192); the β-defensins HBD 1 and 2, which are mainly synthesized by epithelial cells (Zhao C, Wang I, Lehrer RI, Widespread expression beta-defensin hBD-1 in human secretory glands and epithelial cells. FEBS Lett. 1996;396:319-322; Harder J, Bartels J, Christophers E, et al., A peptide antibiotic from human skin, Nature. 1997; 387:861), and LL-37, which was first located in granulocytes (Gudmundsson GH, Agerberth B, Odeberg J, et al. The human gene FALL39 and processing of the cathelin precursor to the antibacterial peptide LL-37 in granulocytes. Eur J Biochem. 1996; 238:325-332) but was subsequently found to be expressed in epithelial cells of the skin, lungs, and gut (Froh M, Agerberth B, Ahangari G, et al. The expression of the gene coding for the antibacterial peptide LL-37 is induced in human keratinocytes during inflammatory disorders, J Biol Chem. 1997; 272:15258-15263; Bals R, Wang X, Zasloff M, et al. The peptide antibiotic LL-37/hCAP-18 is expressed in epithelia of the human lung where it has broad antimicrobial activity at the airway surface. Proc Natl Acad Sci USA. 1998; 95:9541-9546; Agerberth B, Grunewald J, Castanos-Velez E, et al. Antibacterial components in bronchoalveolar lavage fluid from healthy individuals and sarcoidosis patients. Am J Respir Crit Care Med. 1999; 160:283-290).

The defensins are cysteine-containing peptides with three intra-chain disulphide bridges folded in a β-sheet structure with amphipathic properties. The cathelicidins are precursor proteins with a conserved cathelin proregion and a variant C-terminal antibacterial domain (Zanetti M, Gennaro R, Romeo D. Cathelicidins: a novel protein family with a common proregion and a variable C-terminal antibacterial domain. FEBS Lett. 1995; 374:1-5). The amphipathic α-helical LL-37 is the only cathelicidin peptide found in humans (Gudmundsson GH, Agerberth B, Odeberg J, et al. The human gene FALL39 and processing of the cathelin precursor to the antibacterial peptide LL-37 in granulocytes. Eur J Biochem. 1996; 238:325-332).

Other amphipathic α-helical proteins that belong to the cathelicidin family in other species are known as CRAMP, SMAP-29, PMAP-37, BMAP-27, BMAP-28.

Human amphipathic α-helical LL-37 has membranolytic activity on the membrane of microorganisms and viruses. Similar to defensins, cathelicidins act as precursor molecules that can release the antimicrobial peptide LL-37 after proteolytic cleavage. Their structural features clearly distinguish them from defensins. The discovery of cathelicidins commenced after the isolation of the antimicrobial peptide BAC-5 from bovine neutrophils, and the finding that they are cleaved from an inactive precursor. The cathelicidin proteins are characterized by a highly conserved N-terminal domain of about 100 amino acid residues. This domain is flanked by a signal peptide domain on its N-terminus and by an antimicrobial peptide region on its C-terminus. LL-37 and its precursor, hCAP-18, are found at different concentrations in very different cell and tissue types and body fluids in humans. LL-37 was first described in leucocytes and testis, but was soon found in a large variety of cells, tissues and body fluids. LL-37 exhibits a broad spectrum of antimicrobial activity against bacteria, fungi and viral pathogens (reviewed in Ulrich H.N. et al., LL-37, the only human member of the catheliciin family of antimicrobial peptides, Biochimica et Biophysica Acta 1758 (2006): 1408-1425). It has also been demonstrated that LL-37 shows a strong binding affinity for lipopolysaccharides (LPS), an effect that has also been described for rabbit CAP-18. The covalent immobilisation of LL-37 on a titanium surface using flexible hydrophilic polyethylene glycol (PEG) spacer and selective N-terminal conjugation of LL-37, resulted in a surface peptide layer which was capable of killing bacteria when being in contact (Gabriel M et al., Bioconjugate Chem., 2006, 17(2): 548-550).

Currently, blood samples are routinely treated by irradiation or detergents for removal or inactivation of microbial pathogens. Decontamination by heat or chemical methods cannot be used for the inactivation of such pathogens in whole blood samples. On the other hand, the use of irradiation may affect the integrity of DNA of blood cells and should therefore be avoided due to the possibility to develop malicious cancer. Furthermore, the application of irradiation can also entirely kill healthy blood cells. Other antimicrobial compositions, such as detergents, can only be used at very low concentrations to avoid unwanted cell destruction.

It is therefore the object of the invention to provide an alternative method and a purification system for the antimicrobial treatment of blood samples by inactivation of viruses and microorganisms, or their pathogenic components.

The solution provided by the present invention comprises in-vitro methods, compositions and systems for the purification of blood samples, and for the prevention and/or treatment of bacterial or viral diseases. The method of the invention comprises the coupling an amphipathic α-helical cathelicidin protein or a biologically active fragment thereof to a hydrophilic solid carrier, followed by incubating the hydrophilic solid carrier and the immobilized α-helical cathelicidin protein or a biologically active fragment thereof with the blood sample. As a result, the decontaminated blood sample can be extracted and is ready for blood donations or blood transfusions. The invention also concerns a purification system for an antimicrobial treatment of blood samples for blood donations. The invention finally comprises a pharmaceutical composition, comprising an immobilized amphipathic α-helical cathelicidin protein or a biologically active fragment thereof coupled to nanoparticles that may be administered as medicament to a human or animal patient. The pharmaceutical composition of the invention is suitable for use in the treatment and/or prevention of a mammalian bacterial, fungal or viral disease, or a microbial infection which stems from contaminated blood samples. Such diseases are infections in the blood, either bacteremia or septicaemia. In more particular, the methods and compositions disclosed herein are suitable for the treatment and/or prevention of a bacterial disease based on Yersinia enterocolitic such as sepsis and septic shock. As such the methods and compositions can be used for systematic screening of blood donations.

The purification methods, the purification systems and the pharmaceutical compositions according to the present invention allow for a systemic application of amphipathic α-helical cathelicidin protein or a biologically active fragment thereof for the removal and/or inactivation of bacteria, fungi or viruses, or their pathogenic toxins (such as LPS). It has been demonstrated that truncated peptides of the amphipathic α-helical cathelicidin protein LL-37 exhibit increased antimicrobial activity (Larrick et al., Human CAP-18: The novel antimicrobial lipopolysaccharide binding protein, Infect. Immuncom. 63 (1995) 1291-1297). A truncated peptide of an amphipathic α-helical cathelicidin protein can therefore be regarded as "biologically active" if it retains its antimicrobial properties against bacteria, fungi or viral pathogens.

A "biologically active fragment" in the context of the present invention thus refers to a fragment or a truncated peptide of an amphipathic α-helical cathelicidin protein or polypeptide which has a different size, i.e. is smaller or larger, than the full length protein or the wild type protein, but still retains its antimicrobial capability for the destruction of bacteria, fungi or viruses, or for the inactivation of their toxins, present in blood samples to be treated. To exhibit their antimicrobial activity, biologically active fragments of the amphipathic α-helical cathelicidin protein contain the highly conserved cathelicidin domain.

In order to destroy bacterial, fungal or viral activity from human blood samples that are to be used for blood donations, the blood sample to be treated are transferred to a purification system of the invention following its collection from a patient. In a subsequent step, the blood sample is brought in contact with the amphipathic α-helical cathelicidin protein or a biologically active fragment thereof coupled to an appropriate solid carrier such as nanoparticles or membranes. Alternatively, the amphipathic α-helical cathelicidin protein or a biologically active fragment thereof can be coupled to nanoparticles and directly injected intravenously into patients to be treated. Preferably, the particles are made of a bio-degradable or bio-compatible material such as absorbable polymers, preferably polycaprolactone and related co-polymers. Within the treated organisms, the active components are cleared from the blood stream via the reticuloendothelial system (RES) by the liver and are eventually degraded.

For an in-vitro, extracorporeal application, amphipathic α-helical cathelicidin peptides are preferably loaded onto columns which are packed with hydrophilic solid carriers that contain immobilized amphipathic α-helical cathelicidin peptides. In a second variant, amphipathic α-helical cathelicidin proteins or truncated peptides thereof can be loaded on modified membranes such as polymer membranes, or solid supported membranes. In a third variant, the amphipathic α-helical cathelicidin peptide or a biologically active fragment thereof is coupled to the hydrophilic solid carrier using a polyethylene glycol (PEG) spacer. The hydrophilic solid carrier is preferably an organic polymer selected from the group consisting of polyurethane, cellulose, sepharose or sephadex, or a pegylated anorganic matrix material such as pegylated silica. For the purification of a blood sample, the hydrophilic solid carrier is preferably comprised of nanoparticles that may be activated in order to be degradable within the organism of a human or animal body when applied as pharmaceutical composition.

Amphipathic α-helical cathelicidin proteins have been identified in a number of different species. The only human protein is the 37-mer LL-37 consisting of the amino acid sequence LLGDFFRKFK-EKIGKEFKRI-VQRIKDFLRN-LVPRTES. The inventors surprisingly found that covalently immobilized amphipathic α-helical cathelicidin peptides such as LL-37 can be used for the elimination of viral pathogens in human blood samples, which has a number of advantages over the known methods for virus inactivation such as irradiation or the use of detergents.

As such, the amphipathic α-helical cathelicidin proteins or biologically active fragments thereof are suitable for the inactivation or removal of viral pathogens such as influenza virus, hepatitis virus, cytomegalovirus (CMV), HIV and other enveloped viruses that may be present in blood samples. Using the methods of the invention, blood samples can be treated by the purification system of the invention when collected for blood donations in order to provide decontaminated blood samples that are free of bacterial or viral pathogens.

Preferred amphipathic α-helical cathelicidin proteins are selected from the group consisting of LL-37, CRAMP, SMAP-29, PMAP-37, BMAP-27, BMAP-28. For the purification of human blood samples, the use of LL-37 of the family of amphipathic α-helical cathelicidin proteins, or a biologically active fragment thereof, is preferred.

LL-37 peptides can be coupled to a hydrophilic material such as polyurethane, cellulose, sepharose or sephadex, using a polyethylene glycol (PEG) spacer. The hydrophilic carrier can be provided in form of nanoparticles or membranes. The utilized hydrophobic materials such as silica or polymers become hydrophilic by PEGylation. The use of a hydrophilic solid carrier is preferred because it minimizes an unspecific adhesion of serum proteins or coagulations factors when being brought into contact with a blood sample. In addition, it also minimizes the attachment of thrombocytes to the carrier.

In a first aspect, cross-linked cellulose particles are used for coupling LL-37 or any other amphipathic α-helical cathelicidin protein or truncated peptides thereof. Preferably, amine-bearing cellulose particles are used and cross-linked with maleimide-PEG-N-hydroxysuccinimidyl ester or m-maleimidobenzoyl-N-hydroxysuccinimidyl ester and further incubated with LL-37 to produce cellulose-LL-37 or cellulose-PEG-LL-37 particles, respectively.

The invention also concerns a purification system for blood samples, comprising an immobilized amphipathic α-helical cathelicidin protein or a biologically active fragment thereof, coupled to a hydrophilic solid carrier. The purification system furthermore comprises an intake for the blood sample such as a port of a column. In a preferred embodiment, the purification system is comprised of a column that is filled with carrier compounds containing immobilized amphipathic α-helical cathelicidin proteins, preferably LL-37, or a biologically active fragment thereof. Preferably the peptides are coupled to a hydrophilic solid carrier by a polyethylene glycol (PEG) spacer. The coupling of LL-37 peptides is enhanced when using modified LL-37, which terminates with cysteine at its N-terminus. N-terminal cysteine is advantageous because it provides a directional coupling of the peptide to the hydrophilic carrier. The use of hetero bifunctional cross-linker with a short chain is also preferred because it allows a direct conjugation and hence immobilisation of LL-37. For an in-vitro application, such as purification by columns, particles with a diameter > 100 µm, preferably between 100 µm and 400 µm are preferred. For an injection in the course of a medical treatment, the pharmaceutical composition contains particles that are able to migrate through blood capillaries. Preferred particles have a diameter < 10 µm, preferably < 5 µm, preferably in a range between 1 and 5 µm.

The use of amphipathic α-helical cathelicidin proteins or peptides in connection with a hydrophilic carrier is suitable for the treatment or prevention of a mammalian bacterial, fungal or viral disease when provided as pharmaceutical composition. The purification system or the pharmaceutical composition according to the invention allows for the elimination or inactivation of viral pathogens present in blood samples taken from patients, or within the human or animal body in the course of a medical treatment. Because blood samples for blood donations may be contaminated with a variety of different viruses, they cannot be applied to patients in untreated form. In order to avoid an infection with viral pathogens, the purification system of the invention provides an effective alternative for the known treatment or screening methods in the prior art. Using the methods and systems of the invention, blood donations can be treated with high efficiency, reliability and at minimum costs.

The following experiments illustrate that LL-37-coupled beads result in a 30-fold reduction of the virus titer in a reaction solution. A similar reduction can be observed using immobilized LL-37 peptides coupled to the hydrophilic carrier via PEG spacer, suggesting that pegylation does not affect functionality of the immobilized LL-37.

In a further experiment, hemolysis was tested in order to assess the effect of immobilized peptides on red blood cells and the potential release of hemoglobin. The results show only a marginal impact on erythrocyte integrity, thus making the systems and compositions of the invention suitable for the treatment of whole blood applications with blood samples taken from human or animal blood.

### Experimental Data

### Preparation and modification of cellulose beads:

In a typical preparation 5 g of dried cellulose was dissolved in dry dimethylacetamide containing 9% lithium chloride by heating to 165°C for 30 min.After cooling, a yellowish viscous solution was yielded. Using a tissue homogenizer, 20 ml of this solution was first emulsified in 20 ml of silicone oil, then with 20 ml water containing 0.5 ml sodium dodecyl sulfate and stirred in additional 60 ml water. Beads were harvested by centrifugation and oil residues were extracted with acetone. Beads were wet-sieved and the fraction ranging from 300-500 µm in diameter were used for subsequent modification.

To a 10 ml suspension of cellulose beads, 10 ml 0.48 NaOH and 1 ml of epichlorohydrin was added and the mixture was reacted for 30 min at 50 °C. After repeated washing with water and centrifugation the activated beads were treated with 20 ml of a 10 % ethylene diamine solution for 4 h at room temperature. Aminated material was subjected to washing/centrifugation steps as mentioned above.

Amine-bearing particles were then reacted with the long chain cross-linker maleimide-PEG-N-hydroxysuccinimidyl ester (Mal-PEG-NHS, MW 3000) or the short chain compound m-maleimidobenzoyl-N-hydroxysuccinimidyl ester (Mal-Phe-NHS) respectively. To 10 ml of aminated beads suspended in phosphate buffer (pH 7.4) 10 mg of Mal-PEG-NHS or 5 mg of Mal-Phe-NHS respectively was added and stirred for 2 h at room temperature, followed by washing with buffer. One mg of solid-phase synthesized LL37 containing a N-terminal cysteine (CLLGDFFRKSKEKIGKEFKRIVQRIKDFLRNLVPRTES (Cys-LL37)) was added to a 10 ml suspension each and reacted for 2 h. Unbound peptide was removed by repeated washing with buffer. This procedure resulted in the production of LL37-conjugated cellulose (Cell-LL37) and PEG-mediated LL37- modified beads (Cell-PEG-LL37).

Batch testing of modified and pristine cellulose beads was performed with mouse Cytomegalovirus (mCMV). One ml suspensions of unmodified cellulose, Cell-LL37 and Cell-PEG-LL37 and mCMV in phosphate buffer (PBS) alone were challenged with 2x10⁵ PFU (plaque forming units) mCMV for 1h at room temperature. The bead suspension was centrifuged and the supernatant subjected to serial dilution. Aliquots were then tested on mouse embryonic fibroblasts (MEFs). Numbers of formed plaques were determined manually and the virus titer expressed as PFU.

**Table 1 - Batch testing**

| **groups** | **pfu** |
|---|---|
| PBS | 6,80E+04 |
| Beads | 5,90E+04 |
| Beads-LL37 | 1,50E+03 |
| Beads-PEG-LL37 | 2,00E+03 |

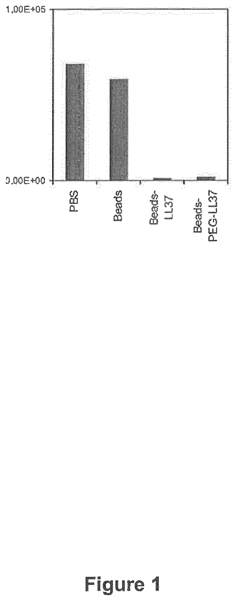

Figure 1 illustrates that a batch-wise treatment of CMV resulted in a reduction of the virus titer of a factor of x40 (LL37) and of appr. x30 (PEG-LL37) respectively. The presence of cellulose beads alone had a minor effect.

Columns were packed by adding appr. 0.5 ml of bead suspension (unmodified cellulose, Cell-LL37, Cell-PEG-LL37) on 1 ml syringes equipped with filter paper frits. A solution of 5x105 PFU mCMV was passed through the column and the eluats were tested the same fashion as described above. Virus in PBS alone served as a control.

**Table 2 - Column Testing**

| **groups** | **Eluat 1** | **Eluat 2** |
|---|---|---|
| PBS | 5,00E+05 | |
| Beads | 1,20E+05 | 2,10E+04 |
| Beads-LL37 | 5,00E+01 | 5,00E+01 |
| Beads-PEG-LL37 | 5,00E+02 | 1,00E+02 |

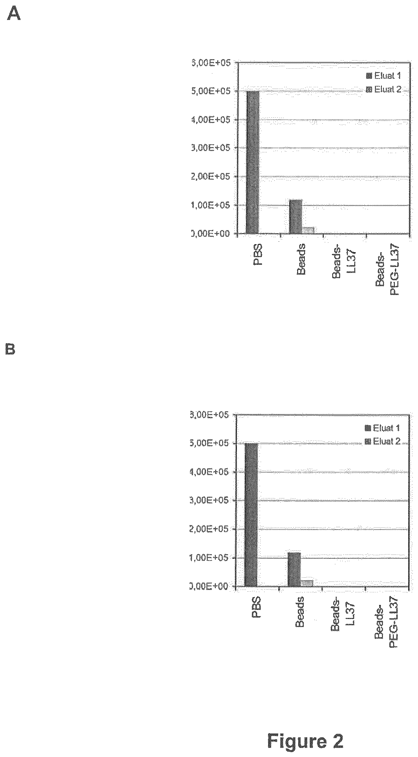

Figure 2 illustrates that the passage of the viruses through the column packed with untreated beads lead to a reduction by a factor x4, suggesting that some adsorption of virus particles occurred. A very pronounced effect was observed on LL37-immobilized beads (reduction by x2400) and on PEG-LL37-modified cellulose (reduction by x240). In Figure 2B the data of Figure 2A are shown in a logarithmic scale.

### Hemolysis assay

In a typical test, 100 µl of particle suspension in PBS, pH 7.4 were mixed with 1000 µl of whole blood, containing 0.109 M Na-citrate and incubated for 45 min at 37°C. The samples were centrifuged and the supernatant was used for photometrical analysis at 450 nm.

**Table 3 Hemolysis assay**

| sample | OD 450 |
|---|---|
| control (PBS) | 1.55 |
| blank (untreated cellulose) | 1.87 |
| LL37 | 1.49 |
| PEG-LL37 | 1.42 |

The results of this experiment indicate that there is only a minor influence of the peptide modification on the release of hemoglobin due to contact of red blood cells and particles. The incubation time used exceeds by far the expected contact time in a column run.

## Claims

1. An in-vitro method for the purification of blood samples, comprising the steps of:
- covalently coupling an amphipathic α-helical cathelicidin protein or a biologically active fragment thereof, which retains its antimicrobial capability for the destruction of bacteria, fungi or viruses, or for the inactivation of their pathogenic toxins, to a hydrophilic solid carrier;
- incubating the hydrophilic solid carrier coupled to the immobilized α-helical cathelicidin protein or the biologically active fragment thereof with a blood sample;
- isolating the treated blood sample.

2. The method according to claim 1, wherein the amphipathic α-helical protein or the biologically active fragment thereof is coupled to the hydrophilic solid carrier by a polyethylene glycol (PEG) spacer.

3. The method according to claim 1 or 2, wherein the hydrophilic solid carrier is comprised of nanoparticles.

4. The method according to claim 1 or 2, wherein the hydrophilic solid carrier is a membrane.

5. The method according to claim 1 or 2, wherein the hydrophilic solid carrier is an organic polymer selected from the group consisting of polyurethane, cellulose, sepharose or sephadex, or a pegylated anorganic matrix material such as pegylated silica.

6. The method according to any one of claims 1 to 5, wherein the amphipathic α-helical cathelicidin protein or the biologically active fragment thereof is selected from the group consisting of LL-37, CRAMP, SMAP-29, PMAP-37, BMAP-27, BMAP-28.

7. The method according to claim 6, wherein the amphipathic α-helical cathelicidin protein or the biologically active fragment thereof is LL-37.

8. The method according to claim 7, wherein amine-bearing cellulose particles are cross-linked with maleimide-PEG-N-hydroxysuccinimidyl ester or m-maleimidobenzoyl-N-hydroxysuccinimidyl ester and further incubated with LL-37 to produce cellulose-LL-37 or cellulose-PEG-LL-37 particles, respectively.

9. A purification system for blood samples, comprising an immobilized amphipathic α-helical cathelicidin protein or a biologically active fragment thereof, which retains its antimicrobial capability for the destruction of bacteria, fungi or viruses, or for the inactivation of their pathogenic toxins, coupled to a hydrophilic solid carrier, and an intake for the blood sample.

10. The purification system according to claim 9, wherein the amphipathic α-helical cathelicidin protein or the biologically active fragment thereof is coupled to the hydrophilic solid carrier by a PEG spacer.

11. The purification system according to claim 9 or 10, wherein the amphipathic α-helical cathelicidin protein or the biologically active fragment thereof is LL-37, which terminates with an N-terminal cysteine.

12. The purification system according to any one of claims 9 to 11, wherein the hydrophilic solid carrier is an organic polymer selected from the group consisting of polyurethane, cellulose, sepharose or sephadex, or a pegylated anorganic matrix material such as pegylated silica.

13. The purification system according to claim 12, wherein the hydrophilic solid carrier is composed of aminated cellulose particles bound to a PEG spacer.

14. A pharmaceutical composition suitable for the treatment and/or prevention of a mammalian bacterial, fungal or viral disease which stems from contaminated blood samples, comprising an immobilized amphipathic α-helical cathelicidin protein or a biologically active fragment thereof, which retains its antimicrobial capability for the destruction of bacteria, fungi or viruses, or for the inactivation of their toxins, covalently coupled to nanoparticles of a hydrophilic solid carrier.

15. The pharmaceutical composition according to claim 14 for use in an antimicrobial treatment and/or prevention of a bacterial, fungal or viral infection or disease.

## Patentansprüche

1. In vitro-Verfahren für die Aufreinigung von Blutproben umfassend die Schritte:
- Kovalentes Ankoppeln eines amphipathischen α-helikalen Cathelicidin-Proteins oder eines biologisch aktiven Fragments davon, welches seine antimikrobielle Fähigkeit zur Zerstörung von Bakterien, Pilzen oder Viren oder zur Inaktivierung deren pathogener Toxine behält, an einen hydrophilen Feststoffträger;
- Inkubieren des an das immobilisierte α-helikale Cathelicidin-Protein oder an das biologisch aktive Fragment davon gekoppelten hydrophilen Feststoffträgers mit einer Blutprobe;
- Isolieren der behandelten Blutprobe.

2. Verfahren nach Anspruch 1, wobei das amphipathische α-helikale Protein oder das biologisch aktive Fragment davon an den hydrophilen Feststoffträger über einen Polyethylenglykol (PEG) Spacer gekoppelt ist.

3. Verfahren nach Anspruch 1 oder 2, wobei der hydrophile Feststoffträger aus Nanopartikeln gebildet ist.

4. Verfahren nach Anspruch 1 oder 2, wobei der hydrophile Feststoffträger eine Membran ist.

5. Verfahren nach Anspruch 1 oder 2, wobei der hydrophile Feststoffträger ein organisches Polymer, ausgewählt aus der Gruppe bestehend aus Polyurethan, Cellulose, Sepharose, Sephadex oder ein pegyliertes anorganisches Matrixmaterial wie pegylierte Kieselsäure ist.

6. Verfahren nach einem der Ansprüche 1 bis 5, wobei das amphipathische α-helikale Cathelicidin-Protein oder das biologisch aktive Fragment davon ausgewählt ist aus der Gruppe bestehend aus LL-37, CRAMP, SMAP-29, PMAP-37, BMAP-27, BMAP-28.

7. Verfahren nach Anspruch 6, wobei das amphipathische α-helikale Cathelicidin-Protein oder das biologisch aktive Fragment davon LL-37 ist.

8. Verfahren nach Anspruch 7, wobei die Amin-tragenden Zellulosepartikel mit Maleimid-PEG-N-hydroxysuccinimidylester oder m-Maleimidobenzoyl-N-hydroxysuccinimidylester kreuzvernetzt sind und weiter mit LL-37 inkubiert werden, um Zellulose-LL-37 beziehungsweise Zellulose-PEG-LL-37-Partikel herzustellen.

9. Aufreinigungssystem für Blutproben, umfassend ein an einen hydrophilen Feststoffträger gekoppeltes immobilisiertes amphipathisches α-helikales Cathelicidin-Protein oder ein biologisch aktives Fragment davon, welches seine antimikrobielle Fähigkeit zur Zerstörung von Bakterien, Pilzen oder Viren oder zur Inaktivierung deren pathogener Toxine behält, und einen Einlass für die Blutprobe.

10. Aufreinigungssystem nach Anspruch 9, wobei das amphipathische α-helikale Cathelicidin-Protein oder das biologisch aktive Fragment davon an den hydrophilen Feststoffträger über einen Polyethylenglykol (PEG) Spacer gekoppelt ist.

11. Aufreinigungssystem nach Anspruch 9 oder 10, wobei das amphipathische α-helikale Cathelicidin-Protein oder das biologisch aktive Fragment davon LL-37 ist, welches mit einem N-terminalen Cystein abschließt.

12. Aufreinigungssystem nach einem der Ansprüche 9 bis 11, wobei der hydrophile Feststoffträger ein organisches Polymer, ausgewählt aus der Gruppe bestehend aus Polyurethan, Zellulose, Sepharose, Sephadex oder ein pegyliertes anorganisches Matrixmaterial wie pegylierte Kieselsäure ist.

13. Aufreinigungssystem nach Anspruch 12, wobei der hydrophile Feststoffträger aus aminierten Zellulosepartikeln besteht, die an einen PEG Spacer gebunden sind.

14. Pharmazeutische Zusammensetzung für die Behandlung und/oder Vorbeugung einer bakteriellen, fungalen oder viralen Erkrankung in einem Säugetier, die von kontaminierten Blutproben stammen, umfassend ein immobilisiertes amphipathisches α-helikales Cathelicidin-Protein oder ein biologisch aktives Fragment davon, was dessen antimikrobielle Fähigkeit zur Zerstörung von Bakterien, Pilzen oder Viren oder zur Inaktivierung von deren Toxinen behält, kovalent gekoppelt an Nano-Partikel eines hydrophilen Feststoffträgers.

15. Pharmazeutische Zusammensetzung nach Anspruch 14 zur Verwendung in einer antimikrobiellen Behandlung und /oder Vorbeugung einer bakteriellen, Pilz- oder Virusinfektion oder -erkrankung.

## Revendications

1. Procédé in vitro de purification d'échantillons de sang, comprenant les étapes consistant à :
- coupler de manière covalente une protéine de cathélicidine à hélice α amphipathique ou un fragment biologiquement actif de celle-ci, laquelle/lequel conserve son pouvoir antimicrobien pour la destruction de bactéries, de champignons ou de virus, ou pour l'inactivation de leurs toxines pathogènes, à un support solide hydrophile ;
- incuber le support solide hydrophile couplé à la protéine de cathélicidine à hélice α immobilisée ou au fragment biologiquement actif de celle-ci avec un échantillon de sang ;
- isoler l'échantillon de sang traité.

2. Procédé selon la revendication 1, dans lequel la protéine à hélice α amphipathique ou le fragment biologiquement actif de celle-ci est couplé au support solide hydrophile par un espaceur de polyéthylèneglycol (PEG).

3. Procédé selon la revendication 1 ou 2, dans lequel le support solide hydrophile est constitué de nanoparticules.

4. Procédé selon la revendication 1 ou 2, dans lequel le support solide hydrophile est une membrane.

5. Procédé selon la revendication 1 ou 2, dans lequel le support solide hydrophile est un polymère organique sélectionné parmi le groupe constitué du polyuréthane, de la cellulose, du Sepharose ou du Sephadex, ou un matériau de matrice inorganique PEGylé tel que de la silice PEGylée.

6. Procédé selon l'une quelconque des revendications 1 à 5, dans lequel la protéine de cathélicidine à hélice α amphipathique ou le fragment biologiquement actif de celle-ci est sélectionné parmi le groupe constitué du LL-37, du CRAMP, du SMAP-29, du PMAP-37, du BMAP-27, du BMAP-28.

7. Procédé selon la revendication 6, dans lequel la protéine de cathélicidine à hélice α amphipathique ou le fragment biologiquement actif de celle-ci est le LL-37.

8. Procédé selon la revendication 7, dans lequel des particules de cellulose porteuses d'aminé sont réticulées avec un ester de maléimide-PEG-N-hydroxysuccinimidyle ou un ester de m-maléimidobenzoyl-N-hydroxysuccinimidyle et sont en outre incubées avec le LL-37 pour produire des particules de cellulose-LL-37 ou de cellulose-PEG-LL-37, respectivement.

9. Système de purification d'échantillons de sang, comprenant une protéine de cathélicidine à hélice α amphipathique immobilisée ou un fragment biologiquement actif de celle-ci, laquelle/lequel conserve son pouvoir antimicrobien pour la destruction de bactéries, de champignons ou de virus, ou pour l'inactivation de leurs toxines pathogènes, couplé à un support solide hydrophile, et une entrée pour l'introduction de l'échantillon de sang.

10. Système de purification selon la revendication 9, dans lequel la protéine de cathélicidine à hélice α amphipathique ou le fragment biologiquement actif de celle-ci est couplé au support solide hydrophile par un espaceur de PEG.

11. Système de purification selon la revendication 9 ou 10, dans lequel la protéine de cathélicidine à hélice α amphipathique ou le fragment biologiquement actif de celle-ci est le LL-37, lequel se termine par une cystéine N-terminale.

12. Système de purification selon l'une quelconque des revendications 9 à 11, dans lequel le support solide hydrophile est un polymère organique sélectionné parmi le groupe constitué du polyuréthane, de la cellulose, du Sepharose ou du Sephadex, ou un matériau de matrice inorganique PEGylé tel que de la silice PEGylée.

13. Système de purification selon la revendication 12, dans lequel le support solide hydrophile est composé de particules de cellulose aminées liées à un espaceur de PEG.

14. Composition pharmaceutique adaptée pour le traitement et/ou la prévention d'une maladie bactérienne, fongique, ou virale présente dans des échantillons de sang contaminés de mammifère, comprenant une protéine de cathélicidine à hélice α amphipathique immobilisée ou un fragment biologiquement actif de celle-ci, laquelle/lequel conserve son pouvoir antimicrobien pour la destruction de bactéries, de champignons ou de virus, ou pour l'inactivation de leurs toxines, couplé de manière covalente à des nanoparticules d'un support solide hydrophile.

15. Composition pharmaceutique selon la revendication 14, destinée à être utilisée dans un traitement antimicrobien et/ou pour une prévention d'une infection ou d'une maladie bactérienne, fongique, ou virale.
